# NEW EUROPEAN PATENT SPECIFICATION

(11) **EP 2 687 103 B2**
(45) Date of publication and mention of the opposition decision: **24.07.2024**
(45) Mention of the grant of the patent: 22.05.2019
(21) Application number: 12177364.2
(22) Date of filing: 20.07.2012
(51) Int. Cl.: A23L 29/10, A23L 5/44, A61K 47/14, A61K 47/22, A61K 47/26, A61K 47/36, A61K 9/107, A61K 31/035, A61K 31/11, A61K 31/232, A23K 20/179, A23L 33/105, A61K 31/07

(54) **Process for manufacturing an aqueous transparent oil-in-water emulsion comprising a carotenoid and emulsion produced**
Verfahren zur Herstellung einer wässrigen transparenten Öl-in-Wasser-Emulsion umfassend ein Carotinoid und hergestellte Emulsion
Procédé de fabrication d'une émulsion d'huile dans l'eau aqueuse transparente comprenant un caroténoïde et émulsion produite

(43) Date of publication of application: 22.01.2014
(73) Proprietor: BASF SE, 67056 Ludwigshafen am Rhein (DE)
(72) Inventor: HELGASON, Thrandur, 67063 Ludwigshafen (DE); KÖPSEL, Christian, 69469 Weinheim (DE); SAMBALE, Clemens, 67459 Böhl-Iggelheim (DE); HABICH, Andreas, 67346 Speyer (DE); HASSE, Andreas, 67256 Weisenheim/Sand (DE)

(56) References cited:
- EP-A1- 0 966 889
- EP-A2- 0 239 086
- WO-A1-2012/059286
- US-A1- 2010 120 713

## Description

The invention relates to a process for manufacturing an aqueous transparent oil-in-water emulsion comprising a carotenoid and to emulsion produced according to said process.

Emulsions containing carotenoids are well known in the states of the art.

The WO 2012/059286 A1 discloses emulsions comprising octenyl-succinic anhydride-modified gum acacia and carotenoids.

The EP 0 966 889 A1 discloses a powder composition which comprises droplets of a fat soluble vitamin which droplets average about 70 to about 200 nm in diameter, and which are dispersed in a modified polysaccharide matrix, beverages and tablets containing the composition.

The US 2010/120713 A1 discloses compositions for the enrichment, fortification and/or coloration of food, beverages, animal feed, cosmetics or pharmaceutical compositions comprising at least one modified starch, β-carotene and optionally at least an adjuvant and/or an excipient

Carotenoids are used as coloring material and as active substances for the human food and animal feed industry as well as in the pharmaceutical sector. In this area they are also used in the last years to replace synthetic azo dyes, otherwise known as the 'Southampton Six': sunset yellow (E110), quinoline yellow (E104), carmoisine (E122), allura red (E129), tartrazine (E102), and Ponceau 4R (E124). These might have negative effects, especially an adverse effect on activity and attention in children. In the past it was difficult to replace the azo dyes by carotenoids and to maintain exactly the same color match of the products as with azo dyes. Carotenoids are insoluble in water. Additionally, they show a great sensitivity to oxidation. Therefore it is difficult to use the carotenoids in crystalline form which is not stable during storage. Generally, a lot of additives are necessary in order to obtain stable carotenoid emulsion, where the carotenoid is protected from oxidation and the emulsion have an improved coloration.

One approach to overcome the problems in handling carotenoids is the manufacturing of micro-emulsions. These micro-emulsions exhibit a soapy taste caused by the used emulsifiers. Emulsifiers, especially ester of long-chain fatty acid with ascorbic acid, in particular ascorbyl palmitate, are also used according to the teaching of US 4,844,934 to obtain an emulsion stable to creaming.

Formation of small emulsions, made by dispersion a liquid oil such as Vitamin E acetate into water phase with a emulsifier followed by homogenization at high pressure requires high amounts of energy with high amount of emulsifier (these are common equipment used for emulsion formation) because the oil phase is liquid at room temperature and the interfacial tension between Vitamin E acetate oil and the water phase is lower than regular vegetable oils (e.g. sunflower oil). On the other hand, it is very much more difficult to emulsify carotenoids because of their low oil solubility and very low water solubility. Furthermore, preventing recrystallization of the carotenoids requires that the ratio of cis vs. trans isomers will be finely tuned to achieve a stable system. Therefore, emulsion containing carotenoids is in fact a different system than emulsion containing pure Vitamin E acetate. Finally, emulsion droplet containing only oil soluble vitamin (such as Vitamin E acetate) cannot be used as colorant.

It is object of the present invention to put a process for manufacturing aqueous carotenoid emulsions at disposal which leads to an emulsion showing improved color, preferably at high carotenoid concentrations, with improved bio-availability of the carotenoids. Additionally, the emulsion obtained by the method according to the invention has to be clear and transparent, meaning a very low turbidity. A further object of the present invention is to use exclusively naturally renewable raw material or naturally identically raw materials. Furthermore, the emulsion has to be producible in an easy and effective way within only few steps. It is further object of the present invention to put a carotenoid containing aqueous emulsions or a carotenoid containing powder as colorant at disposal which replace azo dyes and replicates the visual appearance of the end product colored with azo dyes. Additionally, is object of the invention to put aqueous carotenoid emulsions at disposal which do not contain any azo dyes but replicates the visual appearance of a product colored with azo dyes.

These problems are solved by the process for manufacturing the emulsion according to claim 1 and the emulsions obtained by said processes.

In one embodiment, the emulsion manufactured by the process according to the present invention is an aqueous transparent oil-in-water emulsion comprising an emulsified carotenoid in a concentration of 0.025-2000 ppm, preferably 0.1-200 ppm, 1-30 ppm, 3 - 20 ppm, more preferably 1-15 ppm, 3 - 15 ppm, especially 3-6 ppm. In one embodiment the concentration of the emulsified carotenoid is 5 ppm. In one embodiment, 1 ppm is 1 part per weight in 1 million parts per weight. For the purpose of this invention, an oil-in-water emulsion comprising an emulsified carotenoid means an emulsion of oil-droplets in water, whereby the carotenoid is present in the droplets and solved in the oil.

According to the present invention, the term aqueous transparent oil-in-water emulsion means a clear emulsion with a low turbidity. The turbidity is measured with NTU which is nephelometric turbidity units that are measured with any standard turbidimeter, in one embodiment of the invention with HACH 2100AN Turbidimeter. For the purpose of this invention, a transparent emulsion is defined as an aqueous emulsion containing 5 ppm carotenoid with a NTU value below 35. In one embodiment of the invention, the aqueous emulsion containing 5 ppm has a turbidity value of 1 - 35 NTU, preferably 10 - 35 NTU, more preferably 15 - 30 NTU or especially 15 - 25 NTU.

In a further embodiment, the aqueous emulsion manufactured by the process according to the invention containing 5 ppm carotenoid has a turbidity value selected from the group consisting of: 15, 16, 17, 18, 19, 20, 21, 22, 23, 24 and 25 NTU.

In one alternative of the invention, the carotenoid is selected from the group consisting of: beta-carotene, cantaxanthin, astaxanthin, lutein, zeaxanthin, beta-zeacaroten, lycopene, apocarotenal, bixin, paprika olioresin, capsanthin and capsorubin, preferably apocarotenal, more preferably beta-apo-8-carotenal, All carotenoids can be natural or nature identical. Nature identical carotene is a synthetic carotene which has exactly the same chemical structure as natural carotene found in nature.

The emulsion manufactured by the process according of the present invention has an oil droplet size (using dynamic light scattering) of 100 - 150 nm, especially 130 - 140 nm. According to the invention, the oil droplet size is the z-average size, measured in one embodiment using a Malvern zetasizer nano-s (serial nr. MAL500613). The emulsion manufactured by the process according to the present invention has in one alternative polydispersity index (PDI) between 0.01 and 0.4, preferably between 0.01 and 0.3, more preferably between 0.01 and 0.2, especially between 0.01 and 0.1. Polydispersity index (PDI) defines the width of the oil droplet size distribution and is defined in ISP13321 part 8. The emulsion manufactured by the process according to the present invention has in one alternative 70% of its oil droplets between 0 and 200 nm, preferably 80% of its oil droplets between 0 and 200 nm, more preferably 90% of its oil droplets between 0 and 200 nm, especially 95% of its oil droplets between 0 and 200 nm. In one embodiment, the emulsion manufactured by the process according to the invention has a color intensity value (E 1/1) of 140 - 280, preferably 150 - 250, more preferably 150 - 230, especially 160 - 230. The color intensity value of aqueous solutions (E 1/1) is defined as the absorbance of light at maximum absorbance (different for each carotenoid) going through 1 cm cuvette containing 1% carotenoid emulsion. If the color intensity value (E 1/1) is measured at lower concentration than 1%, the measured value of the color intensity has to be corrected with a dilution factor. E1/1 = (Aₘₐₓ × 20)/(weight of sample (g))

In one embodiment of the emulsion manufactured by the process according to the present invention the carotenoid is melt and/or solved and/or isomerized from trans to cis in triacylglycerol oil, such as MCT oil (medium-chain triacylglycerol), olive oil, corn oil, sunflower oil, peanut oil, soy oil or other alternative vegetable oils.

Accordingly, the emulsion manufactured by the process according to the invention comprises triacylglycerol oil. It may be selected from the group consisting of: MCT oil (medium-chain triacylglycerol), olive oil, sunflower oil, peanut oil, soy oil and vegetable oil, preferably MCT oil in a concentration of 0.1-150 ppm, preferably 1-75 ppm more preferably 5-50 ppm, especially 15-30 ppm.

The triacylglycerol oil used in the present invention is in one embodiment an ester of glycerol where glycerol is esterified to a fatty acid where the fatty acid can have 4-22 carbon chain length and double bond on any of the carbon positions. Preferably, MCT oil is used, where the MCT is a ester of glycerol where glycerol is esterified to a fatty acid where the fatty acids are saturated and have 6-10 carbon chain length, preferably 8 - 10 carbon chain length. The isomerization is carried out for an adequate time at a temperature of 100 - 200°C, preferably 120 - 180°C, more preferably 130 - 160°C, till the trans-isomer content is between 30 and 100%, preferably 30 and 90%, more preferably 35 and 80%, especially 40 and 75%.

The isomerization can be carried out in the presence of an oil soluble antioxidant.

Accordingly, in one embodiment of the invention, the emulsion comprises a fatrespectively an oil-soluble antioxidant in a concentration of 0.001-60 ppm, preferably 0.01-30 ppm, more preferably 0.1-20 ppm, especially 0.5-12 ppm. According to one embodiment of the present invention, oil-soluble antioxidants are soluble in non-polar solvents and form a molecular dispersion.

Oil-soluble antioxidants according to the invention are selected from the group consisting of: alpha, beta, gamma, and delta Tocopherol or mixtures comprising at least two of the tocopherols, whereby the oil soluble antioxidant is in a concentration of 0.001-60 ppm, preferably 0.1-30 ppm, more preferably 1-20 ppm especially 6-12 ppm.

By using the process of the present invention, the carotenoid shows a high stability solved in the triacylglycerol oil, so that no solid particles are within the droplets. The droplets are a mixture of carotenoid, oil-soluble antioxidant and triacylglycerol oil.

The mixture of carotenoid, oil-soluble antioxidant and triacylglycerol oil is a solution (meaning a molecular dispersion of the carotenoid, oil-soluble antioxidant and triacylglycerol oil).

Also disclosed are mixtures wherein the carotene can be crystallized or partly crystallized.

The emulsion manufactured by the process according to the invention comprises modified starch; preferably an octenyl succinate starch.

The modified starch has a concentration of 0.5-600 ppm, preferably 2-300 ppm, more preferably 20-200 ppm, especially 60-120 ppm. The above mentioned polymers have a molecular weight MW-distribution of 10.000 - 2.000.000 g/mol, preferably 20.000 - 1.000.000 g/mol, more preferably 30.000 - 500.000 g/mol.

In a further embodiment, the emulsion manufactured by the process according to the invention comprises a carbohydrate selected from the group comprising mono-, di- and oligosaccharides, glucose syrup, maltose and trehalose, preferably glucose syrup, maltose and trehalose, more preferably glycose syrup and said carbohydrates have a DE (dextrose equivalent) between 20 and 50, preferably between 35 and 50 especially between 43 and 48. The saccharides contains glucose, fructose, galactose or mannose.

Said carbohydrate has a concentration of 0.5-10000 ppm, preferably 2-5000 ppm, more preferably 20-3000 ppm, especially 60-2000 ppm.

The emulsion manufactured by the process according to the present invention comprises a water-soluble antioxidant which is sodium ascorbate whereby the water soluble antioxidant is in a concentration of 0.001-60 ppm, preferably 0.1-30 ppm, more preferably 1-20 ppm, especially 6-12 ppm.

According to one embodiment of the present invention, water-soluble antioxidant forms a molecular dispersion in water.

Subject matter of the present invention is a process for manufacturing the emulsion consisting of the following steps:
a) manufacturing a solution comprising modified starch in a concentration of 0,7 - 70 %, preferably 1 - 50 %, more preferably 5 - 30 %, especially 15 -25 %, sodium ascorbate in a concentration of 0.001 - 10 %, at least one sugar in a concentration of 0,001 - 80 %, preferably 10 - 70 %, more preferably 15 - 60 %, especially 30 - 50 % and water, wherein the solution is obtained by dissolving the components modified starch, sodium ascorbate and the at least one sugar in water,
b) manufacturing a solution by mixing at least one carotenoid in a concentration of 0,1 - 15 %, preferably 0,5 - 5 %, more preferably 0.5 - 3 %, especially 1 - 2 %, adding tocopherol in a concentration of 0.001 - 10 %, preferably 0.001 - 5 %, in triacylglycerol oil in a concentration of 1 - 30 %, preferably 3 - 20 %, more preferably 3 - 10 %, especially 3 - 5 %, and melting the dispersed carotenoid, and optionally isomerizing from trans to cis said carotenoid, at a temperature of 100 - 200°C, 120 - 180°C, more preferably 130 - 160°C,
c) introducing the solution of step b) into solution of step a),
d) performing at least one step of pre-emulsification by passing the mixture of step c) through a rotator/stator mixer,
e) performing at least one step, preferably two steps of high pressure homogenization preferably at a pressure of 300 - 2.000 bar,
f) spray drying the emulsion of step e) to obtain a powder,
g-1) manufacturing an emulsion by introducing the powder of step f) in water; whereby the percent are weight percent referring to the emulsion of step c) to e).

A further subject matter is a process for manufacturing an oil-in-water stock-emulsion comprising the steps a) to f) as mentioned above, wherein in step b), the addition of tocopherol is in a concentration of 0.001 - 5%, and wherein in step g-2) an emulsion is manufactured by introducing the powder of step f) in water in a concentration of 10% - 70%, preferably 10% - 60%, more preferably 20% - 50%, whereby the percent are weight percent referring to the concentration of the powder according to step f) in water.

In the process of the invention, the water soluble antioxidant in a concentration of 0,001 - 10 %, preferably 0,001 - 4 %, more preferably 0,01 - 1.5 %, especially 0.5 - 1.5 % is added in step a).

In the process of the invention, the oil-soluble antioxidant in a concentration of 0,001 - 10 %, preferably 0,001 - 5 %, more preferably 0,01 - 2 %, especially 0.1 - 1 %, in triacylglycerol oil in a concentration of 1 - 30 %, preferably 3 - 20 %, more preferably 3 - 10 %, especially 3 - 5 %, is added in step b).

In one embodiment, the pre-emulsification is carried out till the oil droplet size (z-average size) is 200 nm to 20 µm, preferably 250 nm to 10 µm, more preferably 300 nm to 5 µm. The pre-emulsion is stable for at least 10 hours at 60°C (with stirring), preferably 5 hours at 60°C (with stirring), more preferably 3 hours at 60°C (with stirring).

In one embodiment, a two-stage homogenizer is used in the step of high pressure homogenization. The two-stage homogenizer consists of two pressure valves and therefore each pass though the machine consists of two stages. The first stage with 0-3000 bar, preferably 500-2000 bar, more preferably 650-1500 bar, especially 800-950 bar; and the second stage with 0-3000 bar, preferably 50-2000 bar, more preferably 50-1500 bar, especially 50-1000 bar. In an alternative, the emulsion is passed in one stage through a microfluidizer with 300-2000 bar, preferably 500-1500 bar, more preferably 500-1400 bar. All steps in the process are conducted at temperatures between 0 and 500°C, preferably 20 - 200°C, more preferably 20-200°C especially 50-200°C. The step of high pressure homogenization is carried out in an alternative for at least 2, 3, 4, 5, 6, 7, 8, 9, 10, 15, 20, 30 or even more.

In one embodiment, the process of the invention facilitates the manufacturing of stable emulsion without any surfactants.

Further subject matter of the present invention is the powder comprising a carotenoid according to step f). The powder has in one alternative a residual moisture content of 0-15%, preferably 0.1-10%, more preferably 3-5%.

In one embodiment, the powder produced according to the process of the invention comprises:
- a modified starch in a concentration of 0,7 - 70 %, preferably 1 - 60 %, more preferably 10- 50 %, especially 20 - 40 %,
- the water soluble antioxidant in a concentration of 0,001 - 10 %, preferably 0,001 - 4 %, more preferably 0,001 - 2 %, especially 1 %,
- at least one sugar in a concentration of 0,001 - 80 %, preferably 10 - 80 %, more preferably 20 - 70 %, especially 45 %,
- at least one carotenoid in a concentration of 0,5 - 20 %, preferably 1 - 5 %, more preferably 1 - 3 %, especially 2 -3 %, preferably with a trans-isomer content between 30 and 100%,
- the oil-soluble antioxidant in a concentration of 0,001 - 10 %, preferably 0,001 - 5 %, more preferably 0,01 - 3 %, especially 1 %,
- a triacylglycerol oil in a concentration of 1 - 40 %, preferably 3 - 50 %, more preferably 3 - 15 %, especially 8 %.

In one embodiment of the invention, the powder is obtained by spray drying at inlet temperatures between 80°C and 500°C, preferably 100-300°C, more preferably 100-200°C and an outlet temperature of about 40-150°C, preferably 50-100°C, more preferably 50-90°C.

Another subject matter of the present invention is the (concentrated) emulsion comprising a carotenoid according to step g-2), manufactured by introducing the powder of step f) in water. This (concentrated) emulsion is a stock-emulsion which can be further diluted.

In one embodiment of the invention, the aqueous, transparent emulsion, the concentrated stock-emulsion and/or the powder of the invention are used as colorant, preferably natural or naturally identical colorant. The aqueous emulsion, the concentrated stock-emulsion and/or the powder of the invention are used as colorant in beverages like soft drinks, flavoured water, fruit juices, punches or concentrated forms of these beverages but also alcoholic beverages and instant beverage powders.

In a further embodiment, the aqueous emulsion, the concentrated stock-emulsion and/or the powder of the invention are used in food and/or feed. Typically, the aqueous emulsion, the concentrated stock-emulsion and/or the powder of the invention are used in ice cream, cheese, milk product like milk drinks or yoghurt, soy milk and the like, confectionary products, gums, dessert, candies, puddings, jellies, instant pudding powder, but also in snacks, cookies, sauces, cereals, salad dressing, soups.

The aqueous solution, the concentrated stock-emulsion and/or the powder of the invention can also be used in pharmaceutical preparations, such as tablets or capsules, or cosmetic and dermal products.

In one embodiment, the aqueous emulsion, the concentrated stock-emulsion and/or the powder of the invention are used instead of azo dyes and replace them. The products of the invention replace azo-dyes as food colorant, by replicating the visual appearance of the end product, meaning the final products have the same color and transparency.

### Examples:

### 1. Preparation procedure

a) The modified starch, Na-ascorbic acid and the sugar were dissolved in water having a temperature of 60°C.
b) Apocarotenal, including tocopherol was solved and isomerized in MCT-oil at 130-160°C for 4 minutes.
c) The solution of step b) was mixed with the solution of step a)
d) The mixture of step c) was pre-emulsified in a lab scale rotator/stator mixer for 9 min.
e) The mixture was further emulsified by a high pressure microfluidyzer.
f) The emulsion was spray dried. The inlet temperature was 100 to 120°C. The outlet temperature was about 60°C.
g) A emulsion was manufactured by introducing the dry powder in water at a concentration of carotenoid of 5 ppm.

The powder had residual moisture content in a concentration of 5%.

In the following example the E1/1 value was measured at 460 nm maximum extinction, maximum extinction of 1% apocarotenal in solvent was 2640. Furthermore, absorbance at 600 nm is an indication of turbidity.

### 2. Increasing Apocarotenal concentration

An emulsion according to example 1 was manufactured with the following compounds and concentration, whereby the mixture of step 1 e) was further emulsified by a high pressure microfluidyzer (1 pass at 1000 bar):

| | 2% apocarotenal | 3% apocarotenal | 4% apocarotenal | 5% apocarotenal |
|---|---|---|---|---|
| Apocarotenal (%) | 2 | 3 | 4 | 5 |
| MCT oil (%) | 8 | 12 | 16 | 20 |
| Glucose syrup (%) | 48 | 38 | 34 | 30 |
| Modified starch (%) | 40 | 40 | 40 | 40 |
| Na Ascorbate (%) | 1 | 1 | 1 | 1 |
| Tocopherol (%) | 1 | 1 | 1 | 1 |
| Particle size (nm) | 126 | 142 | 148 | 155 |
| Absorbance at 600 nm | 0.016 | 0.03 | 0.05 | 0.06 |
| E1/1 value | 208 | 206 | 204 | 201 |
| Turbidity NTU | 17 | 31 | 33.7 | 34 |

### 3. Using various sugars

An emulsion according to example 1 was manufactured with the following compounds and concentration, whereby the mixture of step 1 e) was further emulsified by a high pressure microfluidyzer (1 pass at 1000 bar):

| Trehalose as sugar | | Glucose syrup as sugar | | Glucidex 47 as sugar | |
|---|---|---|---|---|---|
| Apocarotenal (%) | 2 | Apocarotenal (%) | 2 | Apocarotenal (%) | 2 |
| MCT oil (%) | 8 | MCT oil (%) | 8 | MCT oil (%) | 8 |
| Trehalose (%) | 48 | Glucose syrup (%) | 48 | Glucidex 47 (%) | 48 |
| Modified starch (%) | 38 | Modified starch (%) | 38 | Modified starch (%) | 38 |
| Na Ascorbate (%) | 1 | Na Ascorbate (%) | 1 | Na Ascorbate (%) | 1 |
| Tocopherol (%) | 1 | Tocopherol (%) | 1 | Tocopherol (%) | 1 |
| Particle size (nm) | 140 | Particle size (nm) | 130 | Particle size (nm) | 111 |
| Absorbance at 600 nm | 0.022 | Absorbance at 600 nm | 0.02 | Absorbance at 600 nm | 0.016 |
| E1/1 value | 208 | E1/1 value | 206 | E1/1 value | 207 |

### 4. Using various modified starch concentration

An emulsion according to example 1 was manufactured with the following compounds and concentration, whereby the mixture of step 1 e) was further emulsified by a high pressure microfluidyzer (1 pass at 800 bar):

| | 10% starch | 20% starch | 40% starch |
|---|---|---|---|
| Apocarotenal (%) | 2 | 2 | 2 |
| MCT oil (%) | 8 | 8 | 8 |
| Glucose syrup (%) | 75 | 65 | 45 |
| Modified starch (%) | 10 | 20 | 40 |
| Na Ascorbate (%) | 1 | 0 | 0 |
| Tocopherol (%) | 1 | 1 | 1 |
| Particle size (nm) | 113 | 134 | 131 |
| Absorbance at 600 nm | 0.018 | 0.022 | 0.022 |
| E1/1 value | 206 | 206 | 206 |
| Turbidity NTU | 23.9 | 19 | 18.7 |

### 5. Using various pressures

An emulsion according to example 1 was manufactured with the following compounds and concentration, whereby the mixture of step 1 e) was further emulsified by a high pressure microfluidyzer (1 pass at 600 or 1000 bar):

| | 600 bar | 800 bar | 1000 bar |
|---|---|---|---|
| Apocarotenal (%) | 2 | 2 | 2 |
| MCT oil (%) | 8 | 8 | 8 |
| Glucose syrup % | 48 | 48 | 48 |
| Modified starch (%) | 38 | 38 | 38 |
| Na Ascorbate (%) | 0 | 0 | 0 |
| Tocopherol (%) | 1 | 1 | 1 |
| Particle size (nm) | 150 | 131 | 123 |
| Absorbance at 600 nm | 0.036 | 0.022 | 0.019 |
| E1/1 value | 207 | 206 | 207 |

### 6. Passing 1-5 times through the microfluidyzer

An emulsion according to example 1 was manufactured with the following compounds and concentration, whereby the mixture of step 1 e) was further emulsified by a high pressure microfluidyzer (at 1000 bar):

| | |
|---|---|
| Apocarotenal (%) | 1 |
| MCT oil (%) | 4 |
| Glucose syrup (%) | 49 |
| Modified starch (%) | 40 |
| Na Ascorbate (%) | 2 |
| Tocopherol (%) | 1 |
| Particle size 1 pass (nm) | 150 |
| Particle size 2 pass (nm) | 114 |
| Particle size 3 pass (nm) | 101 |
| Particle size 4 pass (nm) | 104 |
| Particle size 5 pass (nm) | 102 |

## Claims

1. Process for manufacturing an aqueous transparent oil-in-water emulsion comprising an emulsified carotenoid, whereby the carotenoid is present in the droplets and solved in the oil, with an oil droplets size measured as z-average size with dynamic light scattering of 100-150 nm consisting of the following steps:
a) manufacturing a solution comprising modified starch in a concentration of 0,7 - 70 %, sodium ascorbate in a concentration of 0,001 - 10 %, at least one sugar in a concentration of 0,001 -80 % and water,
wherein the solution is obtained by dissolving the components modified starch, sodium ascorbate and the at least one sugar in water,
b) manufacturing a solution by mixing at least one carotenoid in a concentration of 0,1 - 15 %, adding tocopherol in a concentration of 0,001 - 10 %, in triacylglycerol oil in a concentration of 1 - 30 % and melting the dispersed carotenoid at a temperature of 100- 200°C,
c) introducing the solution of step b) into solution of step a),
d) performing at least one step of pre-emulsification by passing the mixture of step c) through a rotator/stator mixer,
e) performing at least one step, preferably two steps of high pressure homogenization,
f) spray drying the emulsion of step e) to obtain a powder,
g-1) manufacturing an emulsion by introducing the powder of step f) in water, whereby the percent are weight percent referring to the emulsion of step c) to e).

2. Process according to claim 1 whereby the carotenoid is selected from the group consisting of: beta-carotene, cantaxanthin, astaxanthin, lutein, zeaxanthin, beta-zeacaroten, lycopen, apocarotenal, bixin, paprika olioresin, capsanthin and capsorubin.

3. Process according to any of the preceding claims comprising a triacylglycerol oil,
wherein the triacylglycerol oil is selected from the group consisting of: MCT oil (medium-chain triacylglycerol), olive oil, sunflower oil, peanut oil, soy oil and vegetable oil, preferably MCT oil.

4. Process according to any of the preceding claims whereby the modified starch is an octenyl succinate starch.

5. Process according to any of the preceding claims comprising at least one carbohydrate, wherein the carbohydrate is selected from the group consisting of: mono-, di- and oligosaccharides, glucose-syrup, maltose and trehalose, preferably glucose-syrup, maltose and trehalose, more preferably glucose syrup, said carbohydrate having a DE between 20 and 50.

6. Process according to any of the proceeding claims whereby the carotenoid has in step c) an isomerized, trans-isomer content between 30 and 100%.

7. Process for manufacturing an oil-in-water stock-emulsion comprising an emulsified carotenoid, whereby the carotenoid is present in the droplets and solved in the oil, with an oil droplets size measured as z-average size with dynamic light scattering of 100-150 nm consisting of the following steps:
a) manufacturing a solution comprising modified starch in a concentration of 0,7 - 70 %, sodium ascorbate in a concentration of 0,001 - 10 %, at least one sugar in a concentration of 0,001 - 80 % and water,
wherein the solution is obtained by dissolving the components modified starch, sodium ascorbate and the at least one sugar in water,
b) manufacturing a solution by mixing at least one carotenoid in a concentration of 0,1 - 15 %, preferably 0.5 - 5 %, more preferably 0.5 - 3 %, especially 1 - 2 %, adding tocopherol in a concentration of 0,001 - 5 %, in triacylglycerol oil in a concentration of 1 - 30 %, and melting the dispersed carotenoid at a temperature of 100 - 200°C,
c) introducing the solution of step b) into solution of step a),
d) performing at least one step of pre-emulsification by passing the mixture of step c) through a rotator/stator mixer,
e) performing at least one step, preferably two steps of high pressure homogenization, whereby the percent are weight percent referring to the emulsion of step c) to e);
f) spray drying the emulsion of step e) to obtain a powder,
g-2) manufacturing an emulsion by introducing the powder of step f) in water in a concentration of 10% - 70%, whereby the percent are weight percent referring to the concentration of the powder according to step f) in water.

8. An aqueous transparent oil-in-water emulsion produced according to a process of claim 1.

9. An emulsion according to claim 8 comprising an emulsified carotenoid in a concentration of 0.025-300 ppm.

10. An emulsion according to claim 8 with a NTU (Turbidity) value at a concentration of 5 ppm of carotenoid of 1 - 35 NTU and/or with a color intensity value (E 1/1) of 140 - 250, whereby the color intensity value (E 1/1) is defined as the absorbance of light at maximum absorbance going through 1 cm cuvette containing 1% carotene dissolved in organic solvent.

11. The powder according to step f) of claim 1 or 7.

12. The oil-in-water emulsion according to step g-2) of claim 7.

13. Use of the aqueous emulsion of claim 12 or the powder of claim 11 as colorant, preferably natural or nature identical colorant in food, feed, pharmaceutical preparations, cosmetic and/or dermal products and/or as replacement for azo dyes.

## Patentansprüche

1. Verfahren zur Herstellung einer wässrigen transparenten Öl-in-Wasser-Emulsion, umfassend ein emulgiertes Carotinoid, wobei das Carotinoid in den Tröpfchen vorhanden ist und in dem Öl gelöst ist, wobei die Öltröpfchengröße, gemessen als z-Mittelgröße mittels dynamischer Lichtstreuung, 100 bis 150 nm beträgt, bestehend aus den folgenden Schritten:
a) Herstellen einer Lösung, die modifizierte Stärke in einer Konzentration von 0,7 bis 70 %, Natriumascorbat in einer Konzentration von 0,001 bis 10 %, mindestens einen Zucker in einer Konzentration von 0,001 bis 80 % und Wasser umfasst,
wobei die Lösung durch Lösen der Komponenten modifizierte Stärke, Natriumascorbat und des mindestens einen Zuckers in Wasser erhalten wird,
b) Herstellen einer Lösung durch Mischen von mindestens einem Carotinoid in einer Konzentration von 0,1 bis 15 %, Zugeben von Tocopherol in einer Konzentration von 0,001 bis 10 % in Triacylglycerinöl in einer Konzentration von 1 bis 30 % und Schmelzen des dispergierten Carotinoids bei einer Temperatur von 100 bis 200 °C,
c) Einbringen der Lösung aus Schritt b) in die Lösung von Schritt a),
d) Durchführen von mindestens einem Schritt zur Voremulgierung, indem die Mischung aus Schritt c) durch einen Rotor/Stator-Mischer geleitet wird,
e) Durchführen von mindestens einem Schritt, vorzugsweise zwei Hochdruckhomogenisierungsschritten,
f) Sprühtrocknen der Emulsion aus Schritt e), um ein Pulver zu erhalten,
g-1) Herstellen einer Emulsion durch Einbringen des Pulvers aus Schritt f) in Wasser, wobei die Prozentwerte Gewichtsprozent sind, die sich auf die Emulsion aus Schritt c) bis e) beziehen.

2. Verfahren nach Anspruch 1, wobei das Carotinoid ausgewählt ist aus der Gruppe bestehend aus: beta-Carotin, Cantaxanthin, Astaxanthin, Lutein, Zeaxanthin, beta-Zeacarotin, Lycopin, Apocarotinal, Bixin, Paprika-Olioresin, Capsanthin und Capsorubin.

3. Verfahren nach einem der vorhergehenden Ansprüche, umfassend ein Triacylglycerinöl, wobei das Triacylglycerinöl ausgewählt ist aus der Gruppe bestehend aus: MCT-Öl (mittelkettigem Triacylglycerin), Olivenöl, Sonnenblumenöl, Erdnussöl, Sojaöl und pflanzlichem Öl, vorzugsweise MCT-Öl.

4. Verfahren nach einem der vorhergehenden Ansprüche, wobei die modifizierte Stärke eine Octenylsuccinatstärke ist.

5. Verfahren nach einem der vorhergehenden Ansprüche, umfassend mindestens ein Kohlenhydrat, wobei das Kohlenhydrat ausgewählt ist aus der Gruppe bestehend aus: Mono-, Di- und Oligosacchariden, Glucosesirup, Maltose und Trehalose, vorzugsweise Glucosesirup, Maltose und Trehalose, bevorzugter Glucosesirup, wobei das Kohlenhydrat ein DE zwischen 20 und 50 aufweist.

6. Verfahren nach einem der vorhergehenden Ansprüche, wobei das Carotinoid in Schritt c) einen Gehalt an isomerisiertem trans-Isomer zwischen 30 und 100 % aufweist.

7. Verfahren zur Herstellung einer Öl-in-Wasser-Vorratsemulsion, umfassend ein emulgiertes Carotinoid, wobei das Carotinoid in den Tröpfchen vorhanden ist und in dem Öl gelöst ist, wobei die Öltröpfchengröße, gemessen als z-Mittelgröße mittels dynamischer Lichtstreuung, 100 bis 150 nm beträgt, bestehend aus den folgenden Schritten:
a) Herstellen einer Lösung, die modifizierte Stärke in einer Konzentration von 0,7 bis 70 %, Natriumascorbat in einer Konzentration von 0,001 bis 10 %, mindestens einen Zucker in einer Konzentration von 0,001 bis 80 % und Wasser umfasst,
wobei die Lösung durch Lösen der Komponenten modifizierte Stärke, Natriumascorbat und des mindestens einen Zuckers in Wasser erhalten wird,
b) Herstellen einer Lösung durch Mischen von mindestens einem Carotinoid in einer Konzentration von 0,1 bis 15 %, vorzugsweise 0,5 bis 5 %, bevorzugter 0,5 bis 3 %, insbesondere 1 bis 2 %, Zugeben von Tocopherol in einer Konzentration von 0,001 bis 5 % in Triacylglycerinöl in einer Konzentration von 1 bis 30 % und Schmelzen des dispergierten Carotinoids bei einer Temperatur von 100 bis 200 °C,
c) Einbringen der Lösung aus Schritt b) in die Lösung von Schritt a),
d) Durchführen von mindestens einem Schritt zur Voremulgierung, indem die Mischung aus Schritt c) durch einen Rotor/Stator-Mischer geleitet wird,
e) Durchführen von mindestens einem Schritt, vorzugsweise zwei Hochdruckhomogenisierungsschritten, wobei die Prozentwerte Gewichtsprozent sind, die sich auf die Emulsion von Schritt c) bis e) beziehen;
f) Sprühtrocknen der Emulsion von Schritt e), um ein Pulver zu erhalten,
g-2) Herstellen einer Emulsion durch Einbringen des Pulvers aus Schritt f) in Wasser in einer Konzentration von 10 % bis 70 %, wobei die Prozentwerte Gewichtsprozent sind, die sich auf die Konzentration des Pulvers gemäß Schritt f) in Wasser beziehen.

8. Wässrige transparente Öl-in-Wasser-Emulsion, die nach einem Verfahren gemäß Anspruch 1 produziert worden ist.

9. Emulsion nach Anspruch 8, umfassend ein emulgiertes Carotinoid in einer Konzentration von 0,025 bis 300 ppm.

10. Emulsion nach Anspruch 8 mit einem NTU (Trübungs)-Wert bei einer Konzentration von 5 ppm Carotinoid von 1 bis 35 NTU und/oder mit einem Farbintensitätswert (E 1/1) von 140 bis 250, wobei der Farbintensitätswert (E 1/1) als Extinktion des Lichtes bei maximaler Extinktion definiert ist, welches durch eine 1 cm Küvette hindurchgeht, die 1 % Carotin gelöst in organischem Lösungsmittel enthält.

11. Pulver gemäß Schritt f) von Anspruch 1 oder 7.

12. Öl-in-Wasser-Emulsion gemäß Schritt g-2) von Anspruch 7.

13. Verwendung der wässrigen Emulsion nach Anspruch 12 oder des Pulvers nach Anspruch 11 als Färbungsmittel, vorzugsweise als natürliches oder naturidentisches Färbungsmittel in Nahrungsmittel, Futtermittel, pharmazeutischen Zubereitungen, Kosmetik- und/oder Dermalprodukten und/oder als Ersatz für Azofarbstoffe.

## Revendications

1. Procédé de fabrication d'une émulsion huile-dans-eau transparente aqueuse comprenant un caroténoïde émulsifié, le caroténoïde étant présent dans les gouttelettes et en solution dans l'huile, avec une taille de gouttelettes d'huile, mesurée comme taille moyenne Z par diffusion dynamique de la lumière, de 100-150 nm, constitué des étapes suivantes :
a) fabrication d'une solution comprenant de l'amidon modifié en une concentration de 0,7-70 %, de l'ascorbate de sodium en une concentration de 0,001-10 %, au moins un sucre en une concentration de 0,001-80 %, et de l'eau, la solution étant obtenue par dissolution des composants amidon modifié, ascorbate de sodium et l'au moins un sucre dans l'eau,
b) fabrication d'une solution en mélangeant au moins un caroténoïde en une concentration de 0,1-15 %, en ajoutant du tocophérol en une concentration de 0,001-10 %, dans une huile de triacylglycérol en une concentration de 1-30 %, et en faisant fondre le caroténoïde dispersé à une température de 100-200°C,
c) introduction de la solution de l'étape b) dans la solution de l'étape a),
d) mise en œuvre d'au moins une étape de préémulsification en passant le mélange de l'étape c) dans un mélangeur rotor/stator,
e) mise en œuvre d'au moins une étape, préférablement deux étapes, d'homogénéisation à haute pression,
f) séchage par pulvérisation de l'émulsion de l'étape e) afin d'obtenir une poudre,
g-1) fabrication d'une émulsion par introduction de la poudre de l'étape f) dans de l'eau,
les pourcentages étant des pourcentages en poids se référant à l'émulsion de l'étape c) à e).

2. Procédé selon la revendication 1, dans lequel le caroténoïde est choisi dans le groupe constitué par : le bêta-carotène, la canthaxanthine, l'astaxanthine, la lutéine, la zéaxanthine, le bêta-zéacarotène, le lycopène, l'apocaroténal, la bixine, l'oléorésine de paprika, la capsanthine et la capsorubine.

3. Procédé selon l'une quelconque des revendications précédentes, comprenant une huile de triacylglycérol, dans lequel l'huile de triacylglycérol est choisie dans le groupe constitué par : une huile MCT (triacylglycérol à chaîne moyenne), l'huile d'olive, l'huile de tournesol, l'huile d'arachide, l'huile de soja et une huile végétale, préférablement une huile MCT.

4. Procédé selon l'une quelconque des revendications précédentes, dans lequel l'amidon modifié est un succinate d'octényle d'amidon.

5. Procédé selon l'une quelconque des revendications précédentes comprenant au moins un glucide, le glucide étant choisi dans le groupe constitué par : les mono-, di- et oligosaccharides, le sirop de glucose, le maltose et le tréhalose, préférablement le sirop de glucose, le maltose et le tréhalose, plus préférablement le sirop de glucose, ledit glucide ayant un DE compris entre 20 et 50.

6. Procédé selon l'une quelconque des revendications précédentes, dans lequel le caroténoïde possède, dans l'étape c), une teneur en isomère trans, isomérisé comprise entre 30 et 100 %.

7. Procédé de fabrication d'une émulsion mère huile-dans-eau comprenant un caroténoïde émulsifié, le caroténoïde étant présent dans les gouttelettes et en solution dans l'huile, avec une taille de gouttelettes d'huile, mesurée comme taille moyenne Z par diffusion dynamique de la lumière, de 100-150 nm, constitué des étapes suivantes :
a) fabrication d'une solution comprenant de l'amidon modifié en une concentration de 0,7-70 %, de l'ascorbate de sodium en une concentration de 0,001-10 %, au moins un sucre en une concentration de 0,001-80 %, et de l'eau, la solution étant obtenue par dissolution des composants amidon modifié, ascorbate de sodium et l'au moins un sucre dans l'eau,
b) fabrication d'une solution en mélangeant au moins un caroténoïde en une concentration de 0,1-15 %, préférablement 0,5 à 5 %, plus préférablement 0,5 à 3 %, notamment 1 à 2 %, en ajoutant du tocophérol en une concentration de 0,001-5 %, dans une huile de triacylglycérol en une concentration de 1-30 %, et en faisant fondre le caroténoïde dispersé à une température de 100-200°C,
c) introduction de la solution de l'étape b) dans la solution de l'étape a),
d) mise en œuvre d'au moins une étape de préémulsification en passant le mélange de l'étape c) dans un mélangeur rotor/stator,
e) mise en œuvre d'au moins une étape, préférablement deux étapes, d'homogénéisation à haute pression,
f) séchage par pulvérisation de l'émulsion de l'étape e) afin d'obtenir une poudre,
g-2) fabrication d'une émulsion par introduction de la poudre de l'étape f) dans de l'eau en une concentration de 10 % à 70 %, les pourcentages étant des pourcentages en poids se référant à la concentration de la poudre selon l'étape f) dans l'eau.

8. Emulsion huile-dans-eau transparente aqueuse produite selon un procédé selon la revendication 1.

9. Emulsion selon la revendication 8, comprenant un caroténoïde émulsifié en une concentration de 0,025-300 ppm.

10. Emulsion selon la revendication 8, ayant une valeur NTU (turbidité) à une concentration de 5 ppm en caroténoïde de 1-35 NTU et/ou ayant une valeur d'intensité de couleur (E1/1) de 140-250, la valeur d'intensité de couleur (E1/1) étant définie comme l'absorbance de lumière à une absorbance maximale traversant une cuvette de 1 cm contenant 1 % de carotène dissout dans un solvant organique.

11. Poudre selon l'étape f) selon la revendication 1 ou 7.

12. Emulsion huile-dans-eau comprenant un caroténoïde selon l'étape g-2) selon la revendication 7.

13. Utilisation de l'émulsion aqueuse selon la revendication 12, ou de la poudre selon la revendication 11, comme matière colorante, préférablement une matière colorante naturelle ou identique au naturel, dans un produit alimentaire, un produit alimentaire pour animaux, des préparations pharmaceutiques, des produits cosmétiques et/ou dermiques et/ou comme remplacement de colorants azoïques.
